**Europäisches Patentamt**

**European Patent Office**

**Office européen des brevets**

(11) Veröffentlichungsnummer: **0 232 445**
**A1**

(12) # EUROPÄISCHE PATENTANMELDUNG

(21) Anmeldenummer: **86101743.2**

(22) Anmeldetag: **12.02.86**

(51) Int. Cl.⁴: **A 61 L 2/18**

(43) Veröffentlichungstag der Anmeldung: **19.08.87**
Patentblatt **87/34**

(84) Benannte Vertragsstaaten: **AT BE CH DE FR GB IT LI LU NL SE**

(71) Anmelder: **Heinrich Wöhlk Institut für Contactlinsen GmbH & Co., Postfach 4520, D-2300 Kiel 1 (DE)**

(72) Erfinder: **Polzhofer, Kurt, Dr., Ringenrade 15, D-2314 Schönkirchen (DE)**

(54) Reinigungs- und Desinfektionssystem für harte und weiche Kontaktlinsen.

(57) Reinigungs- und Desinfektionssystem für harte und weiche Kontaktlinsen bei dem abgelagerte Substanzen von der Kontaktlinse entfernt werden. Es finden zwei verschiedene Lösungen Verwendung.

Die erste ist eine 30%-ige wäßrige Harnstofflösung, welche das Ablösen der Ablagerungen von der Fläche der Kontaktlinse bewirkt, und die zugleich eine bakterizide und fungizide Wirkung hat. Die zweite Lösung kommt nach der ersten zur Anwendung und besteht aus einer sterilen physiologischen Kochsalzlösung. Diese Spül-Lösung spült die abgelösten Ablagerungen ab und äquilibriert die Kontaktlinse.

EP 0 232 445 A1

ACTORUM AG

Beschreibung:

Reinigungs- und Desinfektionssystem für harte und weiche Kontaktlinsen

Die vorliegende Erfindung betrifft ein Reinigungs- und Desinfektionssystem für harte und weiche Kontaktlinsen, sowie ein Verfahren zum Reinigen und Desinfizieren mit diesem System.

Kontaktlinsen werden direkt auf der Hornhaut des Auges getragen. Die
Tränenflüssigkeit des Auges enthält neben anorganischen Salzen auch
zahlreiche organische Stoffe wie Proteine und Lipide. Diese Stoffe
lagern sich beim längeren Tragen auf der Kontaktlinse ab, wobei die sich
bildenden Niederschläge sowohl die Sicht behindern als auch den Nährboden für Bakterien bilden können, die zu Augeninfektionen führen.

Es ist deshalb notwendig Kontaktlinsen wenigstens einmal täglich gründlich zu reinigen und zu desinfizieren. Dabei müssen insbesondere die
abgelagerten Proteine, Lipide und Mucine von der Kontaktlinse entfernt
werden. Diese Reinigung und die erforderliche Desinfizierung sollen
möglichst einfach durchführbar sein.

Die Reinigung sogenannter weicher Kontaktlinsen ist besonders kritisch,
da das weiche hydrophile Material der Linse Reinigungsmittel absorbiert
und sogar konzentriert und diese später bei der Auflage auf dem Auge
wieder abgeben kann. Die Kontaktlinse kann dadurch beschädigt oder verformt werden und empfindliche Gewebeabschnitte der Hornhaut können gereizt oder sogar beschädigt werden.

Deshalb ist es im allgemeinen nicht möglich Reinigungsmittel, die bei
harten Kontaktlinsen mit ihren wenig oder nicht absorbierenden Flächen
einsetzbar sind auch zur Reinigung weicher Kontaktlinsen zu verwenden.

Aus der DE-OS 24 17 844 ist ein Verfahren zum Reinigen weicher Kontaktlinsen bekannt, bei dem diese in eine praktisch isotonische wässrige
Lösung gelegt werden, die eine wirksame Protease-Menge enthält. Als
Protease wird vorwiegend das Enzym Papain verwendet. Hier werden also
die störenden Ablagerungen auf enzymatischen Wege aufgelöst.

Die DE-OS 28 54 278 schlägt vor die Lösungswirkung einer Protease enthaltenden Reinigungsmittels durch Zusatz eines nichtionogenen Netzmittels zu verbessern. Dabei soll das Reinigungsmittel in Form von Pulver, Tabletten oder flüssigem Konzentrat vorliegen, das vom Verbraucher in Wasser gelöst bzw. mit Wasser verdünnt wird.

Aus der DE-PS 24 43 147 ist ein Verfahren zur Reinigung von Kunststoff-Kontaktlinsen bekannt, bei dem die zu reinigende Linse zunächst mit einer basischen wässrigen Lösung einer aktiven Sauerstoff abgebenden Perverbindung behandelt, dann in eine saure wässrige Lösung einer aktiven Sauerstoff abgebenden Perverbindung eingebracht und schließlich nach Entfernen aus dieser Lösung mit einem nichtionischen Detergens behandelt und abschließend mit Wasser abgespült wird. Hier werden also die störenden Ablagerungen oxidativ zersetzt und dann abgelöst.

Dieses Verfahren ist recht umständlich, insbesondere da es notwendig ist drei fertige Lösungen zu verwenden. Es hat aber den Vorteil, daß es für weiche und harte Kontaktlinsen anwendbar ist, wenn auch mit Unterschieden im Verfahrensablauf insbes. bezüglich Zeit und Temperatur.

Aus der DE-OS 28 35 652 ist ein Mittel zum Reinigen und Desinfizieren von harten und weichen Kontaktlinsen bekannt, bei dem die störenden Ablagerungen ebenfalls oxidativ zersetzt werden, das jedoch in der Anwendung weniger umständlich ist. Bei diesem Mittel ist eine Per-Verbindung mit Ascorbinsäure oder einem ihrer Salze gemischt, wobei beide Komponenten als Pulver in Tablettenform vorliegen. Eine solche Tablette wird in einem vorbestimmten Volumen Wasser gelöst und die zu reinigende Linse wird in diese Lösung gegeben. Aus der Per-Verbindung wird aktiver Sauerstoff frei, wobei zugleich die Ascorbinsäure eine reduktive Zersetzung der Per-Verbindung beginnt. Innerhalb einer Stunde ist die Lösung deaktiviert. Es genügt zur Reinigung die Kontaktlinse für 5 bis 10 Minuten in der Lösung zu lassen und sie dann mit steriler Kochsalzlösung abzuspülen.

Bei diesem System ist nicht gewährleistet, daß bei kurzer Verweildauer der Kontaktlinse in der aktiven Lösung in oberflächennahen Schichten der ontaktlinse kein Wasserstoffperoxid mehr enthalten ist, so daß nach dem Einsetzen der gereinigten Kontaktlinse in das Auge des Kontaktlinsenträgers eine Reizung des Auges nicht auszuschließen ist.

Es ist nun die Aufgabe der vorliegenden Erfindung ein Reinigungs- und Desinfektionssystem für harte und weiche Kontaktlinsen zu schaffen, das bei einfacher Handhabung Kontaktlinsenablagerungen sicher und schnell entfernt, und das für alle Arten von Kontaktlinsen gut verträglich und zudem reizfrei für das Auge des Kontaktlinsenträgers ist.

Diese Aufgabe wird durch ein Reinigungs- und Desinfektionssystem nach den Merkmalen des Anspruchs 1 gelöst.

Die Ansprüche 2 und 3 geben die Zusammensetzung der in diesem System verwendeten Lösungen an. Anspruch 4 beschreibt eine vorteilhafte Unterbringung der Lösung B und Anspruch 5 einen beispielsweisen Ablauf des Reinigungsverfahrens.

Das Reinigungs- und Desinfektionssystem nach der Erfindung beruht auf der überraschenden Erkenntnis, daß die alleinige Verwendung einer 30%-igen wässrigen Harnstofflösung (Lösung A) genügt die störenden Ablagerungen von einer Kontaktlinse abzulösen. Harnstoff sprengt dabei die zwischen Kontaktlinsenmatrix und den anhaftenden Verbindungen, wie Proteine, Lipide, Mucine vorhandene Wasserstoffbrückenbindung und bewirkt damit eine Ablösung dieser störenden Ablagerungen. Diese können dann mittels der Lösung B weggeschwemmt werden.

Die 30%-ige wässrige Harnstofflösung (Lösung A) zeigt zudem eine sehr gute bakterizide und fungizide Wirkung, so daß sie neben der Ablösung der störenden Ablagerungen von der Kontaktlinse zugleich diese Linse desinfiziert.

Bei der Anwendung des Reinigungs- und Desinfektionssystems nach der Erfindung bei weichen Kontaktlinsen bewirkt die Reinigungs-Lösung A auch eine Quellung des Kontaktlinsenmaterials. Dadurch kann die Reinigungslösung in das Material eindringen und dort ihre Wirkung voll entfalten. Beim nachfolgenden Spülen mit der Lösung B wird die Reinigungslösung A vollständig entfernt. Die Quellung der Kontaktlinse geht dabei reversibel zurück, so daß die Kontaktlinse nach Beendigung des Reinigungs und Desinfizierungsvorganges wieder die ursprünglichen Linsenparameter aufweist.

Es ist besonders vorteilhaft die Spül-Lösung B in einer Druckdose unterzubringen, die als sog. Preßpack-System aufgebaut ist. Hier ist die sterile Lösung B in einem sterilen elastischen Behälter untergebracht, der von Stickstoff-Treibgas umgeben ist. Das Ausfüllen der Lösung B in den Behälter für die zu reinigende Kontaktlinse ist damit ohne Kontaminationsgefahr möglich.

Für die Reinigungslösung A ist eine solche Vorsichtsmaßnahme nicht notwendig, da Harnstoff eine desinfizierende Wirkung hat. Diese Lösung kann deshalb in einer Flasche mit Schraubverschluß aufbewahrt werden.

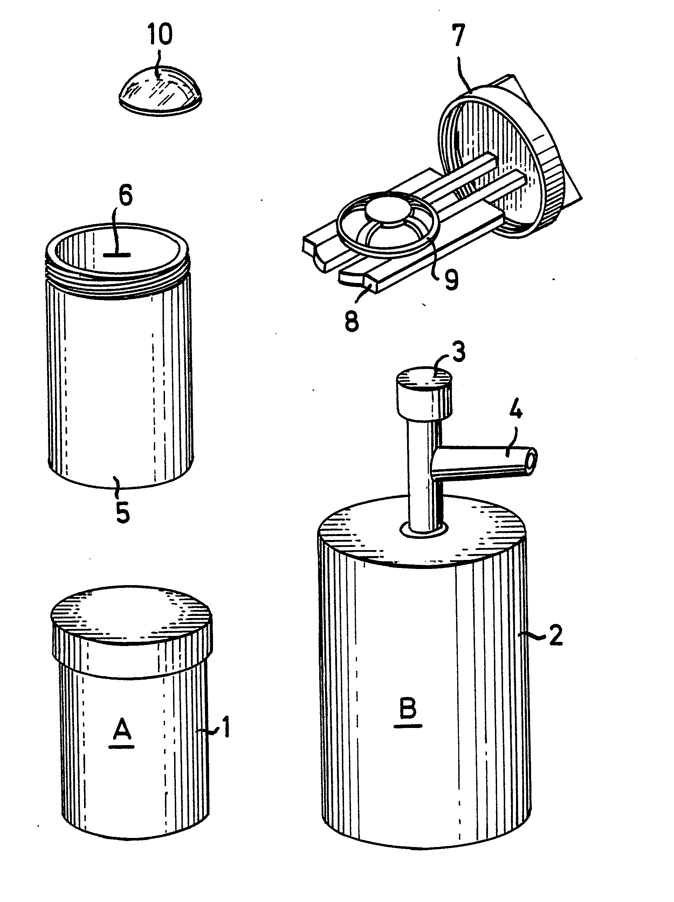

Zur Veranschaulichung der Erfindung wird auf die einzige Figur der beigefügten Zeichnung Bezug genommen, die ein Ausführungsbeispiel eines Reinigungs- und Desinfektions-Sets zeigt.

In dieser Figur ist mit 1 ein Behälter bezeichnet in dem eine 30%-ige wässrige Harnstofflösung, d.h. die Reinigungslösung A aufbewahrt ist. Ein Kilogramm der Lösung A enthält 300 g Harnstoff gelöst in der entsprechenden Menge entmineralisiertem, steril gefiltertem Wasser.

Mit 2 ist eine Druckdose bezeichnet, in der eine sterile physiologische Kochsalzlösung untergebracht ist, die durch Lösen von 9 g Kochsalz in 1000 g aqua purificata hergestellt ist. Diese Spül-Lösung B steht unter dem Druck eines sie umgebenden Treibgases und kann durch Betätigen des Ventiles 3 über die Leitung 4 berührungsfrei ausgefüllt werden.

Mit 5 ist ein Behälter bezeichnet, der mit einem Füllstrich 6 versehen ist. Zu diesem Behälter 5 gehört ein Deckel 7, der mit einem Fortsatz 8 fest verbunden ist. Dieser Fortsatz trägt einen Halter 9 zur Aufnahme der zu reinigenden Kontaktlinse 10.

Zum Reinigen der Kontaktlinse 10 wird diese in den Halter 9 eingesetzt. Darauf wird aus dem Behälter 1 Reinigungs-Lösung A in den Behälter 5 gefüllt und der Deckel 6 wird aufgesetzt. Die Kontaktlinse 10 verbleibt über Nacht in der Lösung A.

Vor dem Einsetzen der Kontaktlinse 10 in das Auge wird die Lösung A aus dem Behälter 5 weggeschüttet. Der Behälter 5 wird sodann aus der Druckdose 2 mit Spül-Lösung B gefüllt. Die Kontaktlinse bleibt 5-10 Minuten in dieser Lösung, die danach abgeschüttet wird.

Abschließend wird der Behälter 5 mit frischer Spül-Lösung B aufgefüllt. Nach Schließen des Deckels 6 wird der Behälter 5 einige Sekunden, ca. 10 Sekunden lang geschüttelt. Danach wird die in der Lösung B äquilibrierte Kontaktlinse 10 aus ihrem Halter 9 entnommen und in das Auge des Benutzers eingesetzt.

Es ist selbstverständlich möglich anstelle nur eines Halters 9 auf der anderen Seite des Fortsatzes einen weiteren solchen Halter vorzusehen, so daß zwei Kontaktlinsen gleichzeitig gereinigt und desinfiziert werden können.

Wie diese Darstellung zeigt ist der Reinigungs- und Desinfizierungsvorgang sehr einfach durchzuführen. Er führt zu einer einwandfrei behandelten, von Rückständen freien und desinfizierten Kontaktlinsen, die in das Auge eingesetzt werden kann, ohne daß Reizungen hervorgerufen werden.

6

Patentansprüche:

1. Reinigungs- und Desinfektionssystem für harte und weiche Kontaktlinsen, gekennzeichnet durch folgende Bestandteile
   a) einer 30%-igen wässrigen Harnstofflösung (Lösung A),
   b) einer sterilen physiologischen Kochsalzlösung (0,9%-ig, Lösung B),
   c) einem Behälter (5) zur Aufnahme der zu reinigenden Kontaktlinse (10) und zum aufeinanderfolgenden Einfüllen der Lösung A und dann der Lösung B.

2. Reinigungs- und Desinfektionssystem nach Anspruch 1, dadurch gekennzeichnet, daß ein Kilogramm der Lösung A 300 g Harnstoff gelöst in der entsprechenden Menge entmineralisiertem, steril gefilterten Wasser enthält.

3. Reinigungs- und Desinfektionssystem nach Anspruch 1, dadurch gekennzeichnet, daß die Lösung B durch Lösen von 9 g Kochsalz in 1000 g aqua purificata hergestellt ist.

4. Reinigungs- und Desinfektionssystem nach Anspruch 1 und 3, dadurch gekennzeichnet, daß die Lösung B ohne Kontaminationsgefahr ausfüllbar in einer Druckdose (2) aufbewahrt ist.

5. Verfahren zum Reinigen und Desinfizieren von harten und weichen Kontaktlinsen unter Verwendung des Systems der Ansprüche 1-4, dadurch gekennzeichnet, daß die zu reinigende Kontaktlinse (10)
   a) mehrere Stunden in der Lösung A gelagert wird, anschließend
   b) 5-10 Minuten in der Lösung B gelagert, und schließlich
   c) 5-10 Sekunden lang in frischer Lösung B geschüttelt wird.

**Europäisches Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 019 600 (SENJU SEIYAKU)<br>* Ansprüche 1,4 * | 1-5 | A 61 L 2/18 |
| | --- | | |
| A | FR-A-2 389 422 (A. RIGOLLOT)<br>* Seite 5, Zeilen 5-10 * | 4 | |
| | --- | | |
| A | EP-A-0 001 888 (FISONS)<br>* Anspruch 8 * | 1 | |
| | --- | | |
| A | US-A-3 939 968 (F.E. RYDER)<br>* Figur 1 * | 1 | |
| | ----- | | |

RECHERCHIERTE SACHGEBIETE (Int. Cl.4)

A 61 L

Der vorliegende Recherchenbericht wurde für alle Patentansprüche erstellt.

| Recherchenort<br>DEN HAAG | Abschlußdatum der Recherche<br>16-06-1986 | Prüfer<br>PELTRE CHR. |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82